# EUROPÄISCHE PATENTANMELDUNG

(11) **EP 4 137 525 A1**
(43) Veröffentlichungstag der Anmeldung: **22.02.2023**
(21) Anmeldenummer: 21192326.3
(22) Anmeldetag: 20.08.2021
(51) Int. Cl.: C08G 18/48, C08G 18/76, C08G 18/82, C08J 11/12, C10B 53/07

(54) **PYROLYSE VON POLYMERER POLYURETHAN-VERBINDUNG ZUR WIEDERGEWINNUNG VON ROHSTOFFEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Für die gewerbliche Ausführung einer Pyrolyse wurde ein Verfahren gemäß Anspruch 1 sowie darin nutzbare Pyrolysevorrichtungen für die Pyrolyse von Pyrolysegut umfassend polymere Verbindung mit mindestens einer Polyurethan-Struktureinheit der Formel (I), 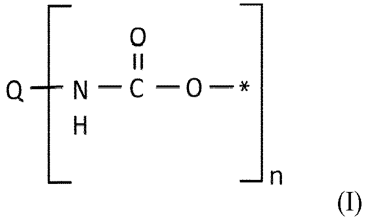 worin
Q für einen Kohlenwasserstoffrest mit 2 bis 8 Kohlenstoffatomen, bevorzugt 3 bis 8 Kohlenstoffatomen, und
n für eine Zahl von 2 bis 4, besonders bevorzugt von 2, steht
und -* eine kovalente Bindung zum Polymerrückgrat bedeutet,
bereitgestellt. Durch dieses Verfahren und die darin nutzbare Pyrolysevorrichtung wird auch bei größeren Mengen von Pyrolysegut eine Menge an Pyrolyseprodukt erhalten, welches für die Synthese von Polyurethan-haltigem Material wiederverwertbare Spaltprodukte enthält.

## Beschreibung

Die Erfindung betrifft ein Pyrolyseverfahren zur thermischen Verwertung von polymerer Polyurethan-Verbindung, eine entsprechende Verwendung einer Pyrolysevorrichtung sowie das Produkt der Pyrolyse, welches solche Rohstoffe enthält, die zur Herstellung von Polyurethan wiederverwertet werden können.

Polyurethan-haltiges Material findet als Polsterung oder als Isoliermaterialien im Kühl- und Baubereich Anwendung. Insbesondere schaumförmige Polyurethan-haltige Materialien werden z.B. als Polster, Matratze, Dämm- oder Isolationsmaterial genutzt. Als Polstermaterial eignet sich Polyurethanweichschaum.

Am Ende der Lebensdauer der Polyurethanmaterial-haltigen Produkte werden sie gegen neue Produkte ausgetauscht und in der Regel entsorgt. Die Gesamtmenge der resultierenden Kunststoffabfälle nimmt jedes Jahr zu. Etwa 60% der Gesamtmenge wird durch Verbrennen und 40% auf der Deponie entsorgt. Bei der Verbrennung wird CO₂ in die Luft emittiert, das zu der globalen Erwärmung beiträgt. Plastikabfall auf den Deponien nimmt wegen seiner geringen Dichte ein großes Volumen ein und kann von dort auch zur allgemeinen Verschmutzung in Flüssen und Meeren beitragen. Aus diesem Grunde ist es wichtig, eine effiziente Recycling Methode zu entwickeln, mit der das Abfallproblem gelöst werden kann und auch gleichzeitig fossile Ressourcen eingespart werden können. Da der Anteil insbesondere der oben genannten Polyurethanweichschäume mit ca. 2 % des Polymermarktes nur einen kleinen Teil ausmacht, stand das Recycling von Polyurethanen zunächst nicht im Fokus der Entwicklungen. Nunmehr ist es jedoch aufgrund des weiter wachsenden Kunststoffmarktes wichtig, für alle Polymerklassen Recycling Technologien zu entwickeln, um sowohl die CO₂ Emission zu vermindern und fossile Energien einzusparen.

Die Verfahren für das Recycling von Kunststoffabfällen lassen sind grob in 3 Kategorien unterteilen:
(1) mechanisches Recycling: Hierbei werden die Kunststoffabfälle so wie sie sind wieder eingeschmolzen und können dann wiederverwendet werden. Dies ist für Polyurethan Elastomere nicht möglich.
(2) chemisches und thermochemisches Recycling: Hierbei werden die Kunststoffabfälle in Monomere depolymerisiert oder zu kleineren Molekülen zersetzt, um nützliche chemische Rohstoffe zu gewinnen und
(3) thermisches Recycling, wobei die Kunststoffabfälle hierin zu Abgas und Wärmeenergie umgesetzt werden.

Die aus dem thermochemischen Recycling gewonnenen chemischen Rohstoffe können zur Synthese neuer Kunstharze oder anderer chemischer Produkte verwertet werden.

Das thermochemische Recycling wird als Pyrolyse bezeichnet. In den meisten Fällen wird die Pyrolyse für Verpackungsmüll angewendet und daraus ein Pyrolyse-Öl gewonnen, das als eine Art recyceltes Naphtha in den bekannten Raffinerie Prozessen mit Cracker als Drop in Lösung verwendet wird. Über die Pyrolyse von Polyurethanen ist wenig bekannt.

Die Verwendung von Katalysatoren oder Additiven kann im Pyrolyse Prozess zur Erniedrigung der Betriebstemperatur, zur Verkürzung der Reaktionszeit, zur Erhöhung der Degradationseffizienz und zur Einschränkung der Produktverteilung führen, was den Prozess effizienter macht.

Garrido et al. (Chemosphere 168 (2017), 667-675) zersetzten Polyurethan-Weichschaum in einem Temperaturfenster von 300 bis 850°C ohne anschließende Kondensation und Isolierung des Pyrolyseproduktes, wobei die Bildung von Isocyanatspezies geräteanalytisch nachgewiesen wurde.

Hileman et al. (J. of Polymer Science, Polymer Chemistry Edition 13 (1975), 571-584) führten die Pyrolyse von kommerziell erhältlichem Polyurethan-Weichschaum bei Temperaturen von 300°C, 500°C, 750°C und 1000°C ohne anschließende Kondensation und Isolierung des Pyrolyseproduktes durch. Dabei wurde bei einer Temperatur von 500°C die größte Bildung von Isocyanatkomponente (hier TDI) beobachtet, welches zu den korrespondierendem Amin hydrolysieren kann. Die thermische Stabilität des TDI und der korrespondierenden Amine ist gemäß Hileman et al. bei einer Temperatur von unter 500°C gegeben, oberhalb einer Temperatur von 750°C zersetzen sich diese zunehmend.

Ravey et al. (J. of Applied Polymer Science, 63 (1997), 47-74) beschreiben die thermische Zersetzung von Polyurethan-Weichschaum im Mikromaßstab bei einer Temperatur von 320 bis 360°C ohne anschließende Kondensation und Isolierung des Pyrolyseproduktes, wobei die Bildung von aromatischem Isocyanat (hier TDI) oder aromatischem Amin (hier DAT) im Gaschromatographen detektiert wird.

Kumagaia et al. (Journal of Analytical and Applied Pyrolysis 126 (2017) 337-345) beschreiben eine bei ca. 800 °C durchgeführte Pyrolyse von Weich und Hartschaum zu Isocyanaten, Diaminen und sehr vielen weiteren Fragmenten aus dem Polyol.

In DE 2410505 C2 wird ein Verfahren zur thermischen Herstellung von Isocyanaten aus Urethanen bei 400°C bis 530 und Unterdruck beschrieben. Versuche mit Polyurethanen unter Normaldruck werden nicht erwähnt.

In DE 2362915 wird die Hydrolyse von Polyurethan Weichschaum zu Diaminen in einen Fließbett vorgeschlagen.

Die im Stand der Technik beschriebenen Pyrolyse-Verfahren eignen sich allesamt nicht für die industrielle bzw. gewerbliche Anwendung, da sie zumeist als Pyrolysen im Microgramm Maßstab für analytische Zecke vorgesehen sind. Die im Stand der Technik dargelegten Pyrolysen werden daher jeweils in nicht für die industrielle bzw. gewerbliche Anwendung geeigneten Pyrolysevorrichtungen ausgeführt.

Insbesondere für die Pyrolyse größerer Mengen an Polyurethan-haltigen Materialien, sind neue, selektivere Verfahren mit passenden Reaktoren nötig, mit deren Hilfe sich durch entsprechende Verfahrensführung die Bildung von ungewünschten Nebenprodukten verringern lässt. Aus Polyurethan-haltigen Materialien soll gezielt Pyrolyseprodukt erhalten werden, das einem hohen Gehalt an für die Polyurethan-Synthese wieder verwertbaren Spaltprodukten, insbesondere aromatische Aminverbindungen wie z.B. Anilin und/oder Toluidin und/oder TDA enthalten.

Es war daher die Aufgabe, für die gewerbliche Ausführung einer Pyrolyse ein Verfahren sowie darin nutzbare Pyrolysevorrichtungen für die Pyrolyse von Pyrolysegut, mindestens umfassend Polyurethan-haltiges Material bereitzustellen, mit dessen Einsatz auch bei größeren Mengen von Pyrolysegut eine Menge an Pyrolyseprodukt erhalten wird, das für die Synthese von Polyurethan-haltigem Material wiederverwertbare Spaltprodukte, bevorzugt aminogruppenhaltige Spaltprodukte in einer Menge von mehr als 10 Gew.-% bezogen auf das Gesamtgewicht an eingesetztem Polyurethan-haltigem Material, enthält. Dabei wird eine Wiedergewinnungsrate von mindestens 40 Gew.-% aminogruppen-haltiger Spaltprodukte bezogen auf das Gesamtgewicht des für die Herstellung der Polyurethan-haltigen polymeren Verbindung eingesetzten organischen Isocyanates.

Ein Gegenstand der vorliegenden Anmeldung ist deshalb ein Verfahren zur Pyrolyse, umfassend mindestens die folgenden Schritte
(a) Einbringung von Pyrolysegut, enthaltend mindestens eine polymere Verbindung mit mindestens einer Polyurethan-Struktureinheit der Formel (I), 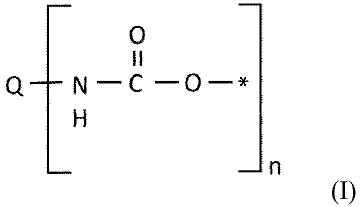 worin
   Q für einen Kohlenwasserstoffrest mit 2 bis 8 Kohlenstoffatomen, bevorzugt 3 bis 8 Kohlenstoffatomen, und
   n für eine Zahl von 2 bis 4, besonders bevorzugt von 2, steht
   und -* eine kovalente Bindung zum Polymerrückgrat bedeutet,
   in einen Reaktor;
(b) Zersetzung zumindest der in Schritt (a) eingebrachten polymeren Verbindung des Pyrolyseguts in dem Reaktor bei einer Temperatur von 200°C bis 350°C, unter Erhalt von in der Gasphase befindlichem Produkt als Pyrolysat und von nicht in der Gasphase befindlichem Pyrolyserückstand, wobei
   (i) während der besagten Zersetzung die Menge an Sauerstoffgas im Reaktor höchstens 2,0 Vol.% bezogen auf das Gesamtvolumen der im Reaktor befindlichen Gase beträgt, und
   (ii) während der besagten Zersetzung das Pyrolysat aus dem Reaktor herausgeführt wird, und
   (iii) der besagte Pyrolyserückstand aus dem Reaktor herausgeführt wird;
(c) Abkühlung des herausgeführten Pyrolysates auf eine Temperatur von weniger als 200°C unter Erhalt von Pyrolyseprodukt, ausgewählt aus Pyrolysatkondensat, Pyrolysatsublimat oder einer Mischung daraus;
(d) optional Aufarbeitung des Pyrolyseprodukts.

Als "Pyrolysegut" wird die Gesamtheit der in den Reaktor zur Pyrolyse eingebrachten Stoffe bezeichnet, die dort in Abwesenheit von Sauerstoffgas oder in Gegenwart einer reduzierten Menge an Sauerstoffgas thermisch behandelt wird. Das Pyrolysegut ist vor der Einbringung in den Reaktor bevorzugt festförmig.

Unter "Pyrolysat" wird die Gesamtheit diejenigen durch Pyrolyse gebildeten Produkte verstanden, die sich unter den Bedingungen des Schrittes (b) (insbesondere als Gas und/oder als Aerosol) in der Gasphase des Reaktors befinden.

Unter "Pyrolyserückstand" wird die Gesamtheit derjenigen durch Pyrolyse gebildeten Stoffe und der sonstigen Rückstände des Pyrolyseguts verstanden, die sich unter den Bedingungen des Schrittes (b) nicht in der Gasphase des Reaktors befinden. Es sind solche Ausführungsformen des Verfahrens bevorzugt, die sich dadurch kennzeichnen, dass der Pyrolyserückstand im Reaktor festförmig oder flüssig ist.

Unter "Pyrolyseprodukt" wird die Gesamtheit derjenigen Produkte aus dem Pyrolysat verstanden, die in Schritt (c) bei der Abkühlung des Pyrolysates durch Kondensation und/oder Resublimation anfallen. Pyrolyseprodukt, das flüssig ist, wird auch als Pyrolyseöl bezeichnet.

Falls nicht explizit im Zusammenhang anders definiert, ist ein Stoff (z.B. Material, Pyrolysegut, Pyrolysat, Pyrolyseprodukt, Pyrolyserückstand) "flüssig", wenn er bei 20°C und 1013 mbar im flüssigen Aggregatzustand vorliegt. Falls nicht explizit im Zusammenhang anders definiert, ist ein Stoff (z.B. Material, Pyrolysegut, Pyrolysat, Pyrolyseprodukt, Pyrolyserückstand) "festförmig", wenn er bei 20°C und 1013 mbar im festen Aggregatzustand vorliegt. Falls nicht explizit im Zusammenhang anders definiert, ist ein Stoff (z.B. Material, Pyrolysegut, Pyrolysat, Pyrolyserückstand) "gasförmig", wenn er bei 20°C und 1013 mbar im als Gas vorliegt.

Ein Stoff ist "organisch", wenn seine chemische Struktur mindestens eine kovalente Kohlenstoff-Wasserstoff-Bindung enthält.

Bei den im Rahmen dieser Anmeldung für Polymere bzw. polymere Inhaltsstoffe angegebenen mittleren Molmassen handelt es sich - sofern nicht explizit anders gekennzeichnet - stets um gewichtsmittlere Molmassen Mw, die grundsätzlich mittels Gelpermeationschromatographie mit Hilfe eines RI-Detektors bestimmbar sind, wobei die Messung zweckmäßig gegen einen externen Standard erfolgt.

Ein "Reaktor" ist ein Volumen, in dem eine chemischen Umwandlung, z.B. eine thermische Zersetzung von Material aus dem Pyrolysegut, stattfindet. Dies kann für eine thermische Zersetzung beispielsweise das Volumen eines beheizten Gefäßes sein, in dem sich das Pyrolysegut befindet.

Es ist erfindungsgemäß vorteilhaft, das Pyrolysegut gemäß erfindungsgemäßem Verfahren in einen Reaktor einzubringen, der sich dadurch gekennzeichnet, dass er ausgewählt wird aus kontinuierlichem Rührkesselreaktor (CSTR), Festbettreaktor, Fließbettreaktor, Schneckenreaktor, Schneckenförderer Reaktor, Flugstromreaktor, Mitreißstromreaktor, Drehrohrreaktor, Fließbettreaktor, Schaufelreaktor. Insbesondere sind solche Reaktoren bevorzugt geeignet, in denen das Pyrolysegut kontinuierlich eingebracht werden kann und werden insbesondere ausgewählt aus Drehrohrreaktor, kontinuierlichem Rührkesselreaktor (CSTR), Festbettreaktor (insbesondere mit kontinuierlichem Bettaustausch (Schachtreaktor) mit innenliegendem Wärmetauscher, bevorzugt mit innenliegenden Wärmetauscherrohren, Schneckenreaktor, Schneckenfördererreaktor, Flugstromreaktor, Drehrohrreaktor oder Wirbelschichtreaktor. Ein ganz besonders bevorzugter Reaktor einer Ausführungsform des Verfahrens wird ausgewählt aus Schneckenreaktor, Drehrohrofen oder Wirbelbett. Weitere für das erfindungsgemäße Verfahren bevorzugte Reaktoren und deren Ausführungsformen werden im Rahmen der Ausführungsformen der erfindungsgemäßen Vorrichtung zur Pyrolyse, sowie im Rahmen einer Ausführungsform des Verfahrens mit Einsatz von Katalysator, beschrieben (*vide infra*)*.*

Das in den Reaktor eingebrachte Pyrolysegut umfasst erfindungsgemäß mindestens eine polymere Verbindung mit mindestens einer Polyurethan-Struktureinheit der Formel (I), 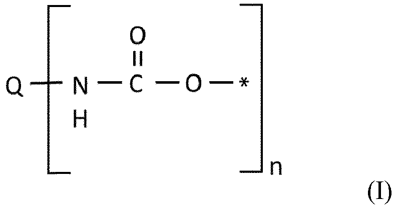 worin
Q für einen Kohlenwasserstoffrest mit 2 bis 8 Kohlenstoffatomen, bevorzugt 3 bis 8 Kohlenstoffatomen, und
n für eine Zahl von 2 bis 4, besonders bevorzugt von 2, steht
und -* eine kovalente Bindung zum Polymerrückgrat bedeutet.

Q leitet sich bevorzugt ab von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohlenwasserstoffeinheiten.

Das erfindungsgemäße Verfahren eignet sich besonders gut für solche Materialien, die eine besagte polymere Verbindung enthalten, welche gemäß Formel (I) eine Gruppe Q mit einem aromatischen Rest aufweist.

Eine ganz besonders bevorzugte Ausführungsform des erfindungsgemäßen Verfahrens ist dadurch gekennzeichnet, dass die polymere Verbindung mindestens eine Struktureinheit der Formel (Ia) enthält 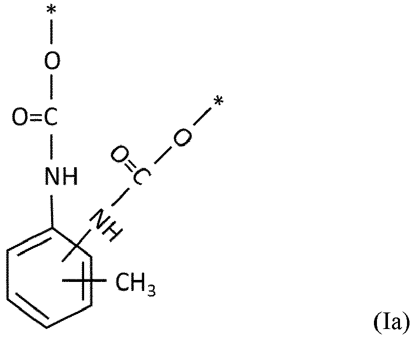 worin
-* für eine kovalente Bindung zum Polymerrückgrat steht.

Verfahren nach einem der vorangehenden Ansprüche, dadurch gekennzeichnet, dass die polymere Verbindung durch

Die in dem besagten Material enthaltene polymere Verbindung kann bevorzugt durch Umsetzung zumindest von
i1) mindestens einer organischen Isocyanatverbindung, enthaltend mindestens zwei bis vier, insbesondere zwei Isocyanatgruppen bindend an eine Kohlenwasserstoffeinheit mit 2 bis 8 Kohlenstoffatomen, bevorzugt mit 3 bis 8 Kohlenstoffatomen; mit
i2) mindestens einer organischen Verbindung mit mindestens zwei Hydroxygruppen, erhalten werden.

Dabei ist es im Rahmen einer weiteren Ausführungsform bevorzugt, wenn die mindestens eine organische Isocyanatverbindung als besagte Kohlenwasserstoffeinheit eine Einheit enthält, die die in i1) genannte Anzahl an Kohlenstoffatomen aufweist und sich ableitet von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohl enwasserstoffeinheiten.

Als besagte organische Isocyanatverbindung wird besonders bevorzugt mindestens eine Verbindung gemäß Formel (II) ausgewählt

Q(NCO)ₙ (II)

worin n für eine Zahl von 2 bis 4, vorzugsweise von 2 bis 3, steht und Q für einen Rest steht, ausgewählt aus einem aliphatischen Kohlenwasserstoffrest mit 2 bis 8 Kohlenstoffatomen, bevorzugt mit 3 bis 8 Kohlenstoffatomen, einem cycloaliphatischen Kohlenwasserstoffrest mit 3 bis 8 Kohlenstoffatomen, einem aromatischen Kohlenwasserstoffrest mit 6 bis 8 Kohlenstoffatomen, bevorzugt mit 10 bis 30 Kohlenstoffatomen oder einem araliphatischen Kohlenwasserstoffrest mit 6 bis 8 Kohlenstoffatomen,.

Enthält besagte polymere Verbindung mindestens eine Struktureinheit der vorgenannten Formel (Ia) Ausführungsform hat es sich als weiter bevorzugte Ausführungsform des Verfahrens erwiesen, wenn in Schritt i1) als mindestens eine organische Polyisocyanatverbindung mindestens ein Diisocyanat der Formel (III) ausgewählt wird,

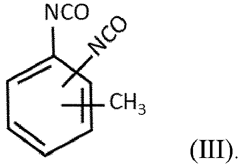

Als bevorzugte Ausführungsform des vorgenannten Schrittes i2) wird mindestens eine organische Verbindung mit mindestens zwei Hydroxygruppen ausgewählt aus Polyesterpolyol, Polyetherpolyol, Polycarbonatpolyol, Polyetheresterpolyol, Polyacrylatpolyol Polyesterpolyacrylatpolyol oder Mischungen daraus, besonders bevorzugt ausgewählt aus der Gruppe der Polyetherpolyole und/oder der Polyesterpolyole.

Die OH-Zahl der eingesetzten organischen Verbindung mit mindestens zwei Hydroxygruppen oder der eingesetzten organischen Verbindungen mit mindestens zwei Hydroxygruppen, jeweils nach DIN 53240-1 (Juni 2013), beträgt bevorzugt von 15 bis 4000 mg KOH/g. Werden mehr als nur eine organische Verbindung mit mindestens zwei Hydroxygruppen verwendet, kann die Mischung aus besagten Verbindungen bevorzugt eine Hydroxylzahl zwischen 20 bis 200 mg KOH/g, insbesondere 25 bis 100 mg KOH/g aufweisen. Die OH-Zahl (auch: Hydroxylzahl) gibt im Falle einer einzelnen zugesetzten organischen Verbindung mit mindestens zwei Hydroxygruppen deren OH-Zahl an. Angaben der OH-Zahl für Mischungen beziehen sich auf die zahlenmittlere OH-Zahl der Mischung, berechnet aus den OH-Zahlen der einzelnen Komponenten in ihren jeweiligen molaren Anteilen. Die OH-Zahl gibt die Menge an Kaliumhydroxid in Milligramm an, welche der bei einer Acetylierung von einem Gramm Substanz gebundenen Menge Essigsäure gleichwertig ist. Sie wird im Rahmen der vorliegenden Ausführungen bestimmt nach der Norm DIN 53240-1 (Juni 2013).

Vorzugsweise weisen die organischen Verbindungen mit mindestens zwei Hydroxygruppen ein zahlenmittleres Molekulargewicht von ≥ 100 g/mol bis ≤ 15000 g/mol, insbesondere ≥ 2000 g/mol bis ≤ 12000 g/mol, bevorzugter ≥ 3500 g/mol bis ≤ 6500 g/mol auf.

Die zahlenmittlere Molmasse Mn (auch: Molekulargewicht) wird im Rahmen dieser Ausführungen durch Gelpermeationschromatographie nach DIN 55672-1 vom August 2007 bestimmt, falls an anderer Stelle nicht explizit anders festgelegt.

Die organischen Verbindungen mit mindestens zwei Hydroxylgruppen weisen eine Funktionalität von 1 bis 8 auf, wobei als "Funktionalität" im Rahmen der vorliegenden Erfindung die theoretische, aus den bekannten Einsatzstoffen und deren Mengenverhältnissen berechnete mittlere Funktionalität (Anzahl an gegenüber Isocyanaten bzw. gegenüber Polyolen reaktiven Funktionen im Molekül) bezeichnet wird.

Verwendbare Polyetheresterpolyole sind solche Verbindungen, die Ethergruppen, Estergruppen und OH-Gruppen enthalten. Vorzugsweise werden organische Dicarbonsäuren mit bis zu 12 Kohlenstoffatomen zur Herstellung der Polyetheresterpolyole eingesetzt, bevorzugt aliphatische Dicarbonsäuren mit 4 bis 6 Kohlenstoffatomen oder aromatische Dicarbonsäuren, die einzeln oder im Gemisch verwendet werden. Beispielhaft seien Korksäure, Azelainsäure, Decandicarbonsäure, Malonsäure, Phthalsäure, Pimelinsäure und Sebacinsäure sowie insbesondere Glutarsäure, Fumarsäure, Bernsteinsäure, Adipinsäure, Phthalsäure, Terephthalsäure und Isophthalsäure genannt. Neben organischen Dicarbonsäuren können auch Derivate dieser Säuren, beispielsweise deren Anhydride sowie deren Ester und Halbester mit niedermolekularen, monofunktionellen Alkoholen mit 1 bis 4 Kohlenstoffatomen eingesetzt werden. Der anteilige Einsatz der oben genannten biobasierten Ausgangsstoffe, insbesondere von Fettsäuren bzw. Fettsäurederivaten (Ölsäure, Sojaöl etc.) ist ebenfalls möglich und kann Vorteile aufweisen, z.B. im Hinblick auf Lagerstabilität der Polyolformulierung, Dimensionsstabilität, Brandverhalten und Druckfestigkeit der Schäume.

Als weitere Komponente zur Herstellung der Polyetheresterpolyole werden Polyetherpolyole eingesetzt, die man durch Alkoxylieren von Startermolekülen wie mehrwertigen Alkoholen erhält. Die Startermoleküle sind mindestens difunktionell, können aber gegebenenfalls auch Anteile höherfunktioneller, insbesondere trifunktioneller, Startermoleküle enthalten.

Startermoleküle sind zum Beispiel Ethylenglykol, Propylenglykol, 1,3-Butandiol, 1,4-Butandiol, 1,5-Pentendiol, 1,5-Pentandiol, 1,6-Hexandiol, 1,7-Heptandiol, 1,8-Octandiol, 1,10-Decandiol, 2-Methylpropan-1,3-diol, Neopentylglykol, 2,2-Dimethyl-1,3-propandiol, 3-Methyl-1,5-pentandiol, 2-Butyl-2-ethyl-1,3-propandiol, 2-Buten-1,4-diol und 2-Butin-1,4-diol, Diethylenglykol, Dipropylenglykol, Glycerin, Trimethylolpropan, di- und trifunktionelle Polyetherpolyole. Die Polyetherpolyole haben bevorzugt eine OH-Funktionalität von 2 bis 4 und ein Molekulargewicht Mn im Bereich von 62 bis 4500 g/mol und insbesondere ein Molekulargewicht Mn im Bereich von 62 bis 3000 g/mol. Auch Startermoleküle mit von OH verschiedenen Funktionalitäten können allein oder in Mischung eingesetzt werden.

Polyetheresterpolyole können auch durch die Alkoxylierung, insbesondere durch Ethoxylierung und/oder Propoxylierung, von Reaktionsprodukten, die durch die Umsetzung von organischen Dicarbonsäuren und deren Derivaten sowie Komponenten mit Zerewitinoffaktiven Wasserstoffen, insbesondere Diolen und Polyolen, erhalten werden, hergestellt werden. Als Derivate dieser Säuren können beispielsweise deren Anhydride eingesetzt werden.

Die Polyesterpolyole können beispielsweise Polykondensate aus mehrwertigen Alkoholen, vorzugsweise Diolen, mit 2 bis 12 Kohlenstoffatomen, vorzugsweise mit 2 bis 6 Kohlenstoffatomen, und Polycarbonsäuren, wie z. B. Di-, Tri- oder sogar Tetracarbonsäuren oder Hydroxycarbonsäuren oder Lactonen sein, bevorzugt werden aromatische Dicarbonsäuren oder Gemische aus aromatischen und aliphatischen Dicarbonsäuren verwendet. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Als Carbonsäuren kommen insbesondere in Betracht: Bernsteinsäure, Glutarsäure, Adipinsäure, Korksäure, Azelainsäure, Sebazinsäure, Decandicarbonsäure, Tetrahydrophthalsäure, Hexahydrophthalsäure, Cyclohexandicarbonsäure, Tetrachlorphthalsäure, Itaconsäure, Malonsäure, 2-Methylbernsteinsäure, 3,3-Diethylglutarsäure, 2,2-Dimethylbernsteinsäure, Dodekandisäure, Endomethylentetrahydrophthalsäure, Dimerfettsäure, Trimerfettsäure, Zitronensäure, Trimellithsäure, Benzoesäure, Maleinsäure, Fumarsäure, Phthalsäure, Isophthalsäure und Terephthalsäure. Verwendet werden können ebenso Derivate dieser Carbonsäuren, wie beispielsweise Dimethylterephthalat. Die Carbonsäuren können dabei sowohl einzeln als auch im Gemisch verwendet werden. Als Carbonsäuren werden bevorzugt Adipinsäure, Sebacinsäure und/oder Bernsteinsäure, besonders bevorzugt Adipinsäure und/oder Bernsteinsäure, verwendet.

Hydroxycarbonsäuren, die als Reaktionsteilnehmer bei der Herstellung eines Polyesterpolyols mit endständigen Hydroxylgruppen mitverwendet werden können, sind beispielsweise Milchsäure, Äpfelsäure, Hydroxycapronsäure, Hydroxybuttersäure, Hydroxydecansäure, Hydroxystearinsäure und dergleichen. Geeignete Lactone sind unter anderem Caprolacton, Butyrolacton und Homologe.

Zur Herstellung der Polyesterpolyole kommen insbesondere auch biobasierte Ausgangsstoffe und/oder deren Derivate in Frage, wie z. B. Rizinusöl, Polyhydroxyfettsäuren, Ricinolsäure, Hydroxyl-modifizierte Öle, Weintraubenkernöl, schwarzem Kümmelöl, Kürbiskernöl, Borretschsamenöl, Sojabohnenöl, Weizensamenöl, Rapsöl, Sonnenblumenkernöl, Erdnussöl, Aprikosenkernöl, Pistazienöl, Mandelöl, Olivenöl, Macadamianussöl, Avocadoöl, Sanddornöl, Sesamöl, Hanföl, Haselnussöl, Primelöl, Wildrosenöl, Distelöl, Walnussöl, Fettsäuren, hydroxylmodifizierte und epoxidierte Fettsäuren und Fettsäureester, beispielsweise basierend auf Myristoleinsäure, Palmitoleinsäure, Ölsäure, Vaccensäure, Petroselinsäure, Gadoleinsäure, Erukasäure, Nervonsäure, Linolsäure, alpha- und gamma-Linolensäure, Stearidonsäure, Arachidonsäure, Timnodonsäure, Clupanodonsäure und Cervonsäure. Insbesondere bevorzugt sind Ester der Rizinolsäure mit mehrfunktionellen Alkoholen, z.B. Glycerin. Bevorzugt ist auch die Verwendung von Mischungen solcher biobasierten Säuren mit anderen Carbonsäuren, z.B. Phthalsäuren.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester. Vorzugsweise verwendet werden Ethylenglykol, Diethylenglykol, 1,4-Butandiol, 1,5-Pentandiol, 1,6-Hexandiol oder Mischungen aus mindestens zwei der genannten Diole, insbesondere Mischungen aus 1,4-Butandiol, 1,5-Pentandiol und 1,6-Hexandiol.

Daneben können auch Polyole wie Trimethylolpropan, Glycerin, Erythrit, Pentaerythrit, Trimethylolbenzol oder Trishydroxyethylisocyanurat eingesetzt werden, wobei Glycerin und Trimethylolpropan bevorzugt sind.

Die als organische Verbidnung mit mindestens zwei Hydroxygruppen verwendbaren Polyetherpolyole werden nach dem Fachmann bekannten Herstellungsmethoden erhalten, wie beispielsweise durch anionische Polymerisation von einem oder mehreren Alkylenoxiden mit 2 bis 4 Kohlenstoffatomen mit Alkalihydroxiden, wie Natrium- oder Kaliumhydroxid, Alkalialkoholaten, wie Natriummethylat, Natrium- oder Kaliumethylat oder Kaliumisopropylat, oder aminischen Alkoxylierungs-Katalysatoren, wie Dimethylethanolamin (DMEA), Imidazol und/oder Imidazolderivate, oder DMC-Katalysatoren unter Verwendung mindestens eines Startermoleküls, das 2 bis 8, vorzugsweise 2 bis 6 reaktive Wasserstoffatome gebunden enthält.

Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Bevorzugte Alkylenoxide sind Propylenoxid und Ethylenoxid, besonders bevorzugt sind Copolymere des Propylenoxids mit Ethylenoxid. Die Alkylenoxide können in Kombination mit CO₂ umgesetzt werden.

Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylendiamin und 2,2'-, 2,4'- und 4,4'-Diaminodiphenylmethan.

Vorzugsweise verwendet werden zwei oder mehrwertige Alkohole, wie Ethandiol, 1,2- und 1,3-Propandiol, Diethylenglykol, Dipropylenglykol, 1,4-Butandiol, 1,6-Hexandiol, Paraformaldehyd, Triethanolamin, Bisphenole, Glycerin, Trimethylolpropan, Pentaerythrit, Sorbit und Saccharose.

Verwendbare Polycarbonatpolyole sind Hydroxylgruppen aufweisende Polycarbonate, zum Beispiel Polycarbonatdiole. Diese entstehen in der Reaktion von Kohlensäurederivaten, wie Diphenylcarbonat, Dimethylcarbonat oder Phosgen, mit Polyolen, bevorzugt Diolen. Beispiele derartiger Diole sind Ethylenglykol, 1,2-und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenole und lactonmodifizierte Diole der vorstehend genannten Art.

Statt oder zusätzlich zu reinen Polycarbonatdiolen können auch Polyether-Polycarbonatdiole eingesetzt werden, welche beispielsweise durch Copolymerisation von Alkylenoxiden, wie zum Beispiel Propylenoxid, mit CO₂ erhältlich sind.

Als Verbindungen mit gegenüber Isocyanaten reaktionsfähigen Wasserstoffatomen können auch Polymerpolyole, PHD-Polyole und PIPA-Polyole als organische Verbindung mit mindestens zwei Hydroxygruppen eingesetzt werden. Polymerpolyole sind Polyole, die Anteile von durch radikalische Polymerisation geeigneter Monomere wie Styrol oder Acrylnitril in einem Basispolyol erzeugten festen Polymeren enthalten. PHD (Polyhydrazodicarbonamid)-Polyole werden beispielsweise hergestellt durch in situ Polymerisation eines Isocyanats oder einer Isocyanat-Mischung mit einem Diamin und/oder Hydrazin (bzw. Hydrazinhydrat) in einem Polyol, bevorzugt einem Polyetherpolyol. Bevorzugt wird die PHD-Dispersion hergestellt durch Umsetzung einer Isocyanat-Mischung aus 75 bis 85 Gew.-% 2,4-Toluylendiisocyanat (2,4-TDI) und 15 bis 25 Gew.-% 2,6-Toluylendiisocyanat (2,6-TDI) mit einem Diamin und/oder Hydrazinhydrat in einem Polyetherpolyol hergestellt durch Alkoxylierung eines trifunktionellen Starters (wie beispielsweise Glycerin und/oder Trimethylolpropan). Bei PIPA-Polyolen handelt es sich um durch Polyisocyanat-Polyaddition mit Alkanolaminen-modifizierte Polyetherpolyole, wobei das Polyetherpolyol bevorzugt eine Funktionalität von 2,5 bis 4,0 und eine Hydroxylzahl von 3 mg KOH/g bis 112 mg KOH/g (Molekulargewicht 500 g/mol bis 18000 g/mol) aufweist.

Es können auch Isocyanat-reaktive Substanzen mit zellöffnender Wirkung eingesetzt werden, wie beispielsweise Copolymere aus Ethylenoxid und Propylenoxid mit einem Überschuss an Ethylenoxid oder aromatischen Diaminen wie Diethyltoluendiamin.

Für die Herstellung von Polyurethanschaumstoffen im Kaltschaumverfahren werden im Rahmen einer weiteren Ausführungsform mindestens zwei Hydroxylgruppen aufweisender Polyether mit einer OH-Zahl von 20 bis 50 mg KOH/g als organische Verbindung mit mindestens zwei Hydroxygruppen eingesetzt, wobei die OH-Gruppen zu mindestens 80 Mol-% aus primärem OH-Gruppen bestehen (Bestimmung mittels 1H-NMR (z.B. Bruker DPX 400, Deuterochloroform)). Besonders bevorzugt beträgt die OH-Zahl 25 bis 40 mg KOH/g, ganz besonders bevorzugt 25 bis 35 mg KOH/g.

Neben der zuvor definierten Struktureinheit der Formel (I) bzw. (Ia) kann die polymere Verbindung des Materials optional zusätzlich Isocyanurat-Struktureinheiten der Formel 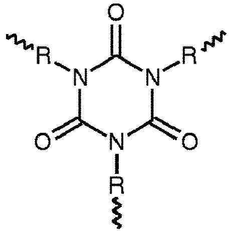 enthalten, worin R für einen bivalenten Kohlenwasserstoffrest, insbesondere für einen bivalenten aromatischen Kohlenwasserstoffrest, steht. Für diese Ausführungsform hat es sich als bevorzugt erwiesen, wenn der Anteil dieser Isocyanurat-Struktureinheiten am Gesamtgewicht des Materials höchstens 1 Gew.-% beträgt.

Bevorzugt handelt es sich bei dem die besagte polymere Verbindung enthaltenden Material um einen Schaum, noch bevorzugter um einen Polyurethanschaum. Wenn das Material in Form eines Polyurethanschaumes vorliegt, handelt es sich wiederum bevorzugt um einen Polyurethanweichschaum oder einen Polyurethanhartschaum. Ein Polyurethanweichschaum ist eine ganz besonders bevorzugte Ausführungsform der in Schritt a) des Verfahrens im Pyrolysegut eingebrachten polymeren Verbindung.

Wenn besagte polymere Verbindung in Form eines Polyurethanweichschaumes vorliegt, hat es sich bevorzugterweise als vorteilhaft erwiesen, wenn der Polyurethanweichschaum nach DIN 7726: 1982-05 bei Druckbelastung bei 10 % Stauchung eine Druckspannung von ≤ 15 kPa aufweist, gemessen nach DIN 53421:1984-06.

Es hat sich als vorteilhaft erwiesen, wenn die besagte polymere Verbindung des Pyrolyseguts bevorzugt in Form festförmiger Teilchen (insbesondere in Form eines körnigen Gemenges) in den Reaktor eingebracht wird. Ein entsprechendes körniges Gemenge wird aus einer Vielzahl an losen, festförmigen Partikeln gebildet, die wiederum sogenannte Körner umfassen. Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen. Die Rieselfähigkeit eines körnigen Gemenges betrifft sein Vermögen, unter eigenem Gewicht frei aus einem Rieseltesttrichter mit einem Auslauf von 16,5 mm Durchmesser zu rieseln.

Vorzugsweise weisen die besagten festförmigen Teilchen, insbesondere die losen, festförmigen Partikel des körnige Gemenges, einen mittleren Durchmesser X₅₀,₃ (Volumenmittel) von 0,01 mm bis 5 cm, bevorzugt von 0,1 mm bis 5 cm, auf. Der mittlere Teilchengrößendurchmesser X_{50,3} wird durch Siebung oder mittels eines Partikelgrößenanalysators Camsizer der Fa. Retsch bestimmt.

Die Dosierung und Prozessierbarkeit des Pyrolysegutes in der Pyrolyse des erfindungsgemäßen Verfahrens lässt sich vereinfachten, indem besagtes Pyrolysegut im Schritt (a) neben besagter polymerer Verbindung zusätzlich mindestens einen Füllstoff enthält. Besagter Füllstoff katalysiert bevorzugt nicht im Rahmen der Pyrolyse die thermische Zersetzung von Polyurethan. Dabei ist es wiederum besonders bevorzugt, wenn der Füllstoff mindestens ein Metalloxid ist, das im Rahmen der Pyrolyse der thermischen Zersetzung von Polyurethan nicht katalytisch wirksam ist, welches bevorzugt ausgewählt aus SiO₂.

Das besagte Material wird vorzugsweise mit einem Füllstoff z.B. Sand gemischt, wodurch eine kontinuierliche Prozessführung des erfindungsgemäßen Verfahrens vereinfacht wird. Insbesondere wird ein Verkleben der besagten polymeren Verbindung im Reaktor und in der Zuführung der Dosiervorrichtung zum Reaktor vermieden. Im Rahmen einer Ausführungsform wird der Füllstoff und die besagte polymere Verbindung im Volumenverhältnis Füllstoff zu besagter polymerer Verbindung von mindestens 0,1 zu 1 bis 10 zu 1 dem Pyrolysegut als Gemisch zugeführt.

Das Pyrolysegut kann zusätzlich mindestens einen Katalysator enthalten. Dieser Katalysator beeinflusst die Reaktion der Zersetzung der besagten polymeren Verbindung. Ein entsprechender Katalysator kann die Pyrolysetemperatur herabsetzen, das Produktspektrum auf gewünschte Produkte reduzieren und gegebenenfalls die Verkokung minimieren. Für einen effizienten Prozess sind kostengünstige Katalysatoren bevorzugt.

Als Katalysatoren können natürlich vorkommende Materialien wie anorganische Salze, feuerfeste Oxide, Mineralien und Industriesteine, in die optional Ionen in einem einfachen Ionenaustauschprozess ausgetauscht werden können, fungieren, da sie keine umfangreiche Synthese erfordern. Sie sind leicht verfügbar und sind daher relativ billig. Synthetische Katalysatoren wie Zeolithe (ZSM-5, ZeolithX,Y, etc) sind dagegen ein Beispiel für Katalysatoren, die zwar effektiv, aber nicht billig sind, da sie speziell hergestellt werden müssen.

Neben der Funktion als Katalysator können Katalysatoren ebenfalls die Dosierung und Prozessierbarkeit des Pyrolysegutes in der Pyrolyse vereinfachen. Bei Einsatz eines Katalysators kann in diesem Fall die eingesetzte Menge an Füllstoff reduziert werden. Im Rahmen einer Ausführungsform wird die Gesamtmenge an Füllstoff und Katalysator im Verhältnis zur Menge des besagten Materials im Volumenverhältnis von mindestens 0,1 zu 1 bis 10 zu 1 dem Pyrolysegut als Gemisch zugeführt.

Das in Schritt (a) in den Reaktor eingebrachte Pyrolysegut wird gemäß Schritt (b) unter Bildung von Pyrolysat und Pyrolyserückstand zumindest teilweise zersetzt.

Das Pyrolysegut wird nach dessen Einbringung in den Reaktor auf eine Temperatur im Bereich von 200°C bis 350°C aufgeheizt. Eine besonders erfolgreiche Verbesserung des Ergebnisses des erfindungsgemäßen Verfahrens lässt sich erreichen, wenn im Rahmen einer Ausführungsform das eingebrachte Pyrolysegut auf 200°C bis 350°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes 1 Sekunde bis 2 Stunden, bevorzugt zwischen 2 Minuten und 60 Minuten, beträgt, und wobei die Temperatur und der Gehalt von Sauerstoffgas im Reaktor während dieses Zeitraums die in Schritt (b) definierten Werte betragen.

Davon unabhängig lassen sich gute Ergebnisse erzielen, wenn die in Schritt (b) stattfindende Herausführung des Pyrolysates aus dem Reaktor durch einen durch den Reaktor geführten Gasstrom oder durch Sog gewährleistet wird und weiter bevorzugt dadurch eine Verweildauer des Pyrolysates als Zeitraum zwischen dem Zeitpunkt der Einbringung des besagten im Schritt (a) in den Reaktor eingebrachten Materials und dem Zeitpunkt der Ausbringung des Pyrolysates 0,1 Sekunden bis 600 Sekunden, bevorzugt zwischen 0,5 Sekunden und 300 Sekunden, besonders bevorzugt 0,5 Sekunden bis 200 Sekunden, beträgt.

Wird ein Gasstrom zu diesem Zweck durch den Reaktor geführt, eignet sich als Gas für diesen Gasstrom insbesondere ein Inertgas, bevorzugt ausgewählt aus Stickstoff, Argon, CO₂, NO oder einem Gemisch daraus.

Wird ein Gasstrom zur Ausbringung des Pyrolysates genutzt, ist es erfindungsgemäß bevorzugt, wenn die Fließgeschwindigkeit des Gasstromes im Reaktor als Leerrohrgeschwindigkeit im Bereich von 0,01 m/s bis 20 m/s liegt. Sollte als Reaktor ein Festbettreaktor gewählt werden, ist es erfindungsgemäß bevorzugt, wenn die Fließgeschwindigkeit des Gasstromes im Reaktor als Leerrohrgeschwindigkeit im Bereich von 0,03 m/s bis 1 m/s liegt. Sollte als Reaktor ein Wirbelbettreaktor gewählt werden, ist es erfindungsgemäß bevorzugt, die Fließgeschwindigkeit des Gasstromes im Reaktor als Leerrohrgeschwindigkeit im Bereich von 0,5 m/s bis 2 m/s liegt. Sollte als Reaktor ein Flugstromreaktor gewählt werden, ist es erfindungsgemäß bevorzugt, die Fließgeschwindigkeit des Gasstromes im Reaktor als Leerrohrgeschwindigkeit im Bereich von 5 m/s bis 20 m/s liegt.

Als erfindungsgemäße Bedingungen für die Zersetzung im Rahmen der Pyrolyse in Schritt (b) beträgt die Temperatur im Reaktor 200°C bis 350°C und die Menge an Sauerstoffgas im Reaktor beträgt von 0 bis 2,0 Vol.% bezogen auf das Gesamtvolumen der im Reaktor befindlichen Gase.

Für die Einstellung der erfindungsgemäßen Menge an Sauerstoffgas, wird der mit besagtem Material des besagten Pyrolysegutes befüllte Reaktor mit Inertgas, insbesondere mit Stickstoff, Argon, CO₂, NO oder einer Mischung daraus befüllt. Dem Inertgas können zusätzlich von Sauerstoffgas verschiedene reaktive Gase beigemischt werden, insbesondere ausgewählt aus Methan, gasförmigem H₂O, Wasserstoffgas oder Gemischen daraus.

Um durch die Einbringung des Pyrolysegutes in den Reaktor das Eintragen von Sauerstoffgas zu minimieren, kann das Pyrolysegut vor dessen Einbringung in Schritt (a) von Sauerstoffgas befreit werden, z.B. durch Austreiben des Sauerstoffgases mittels einer Strippung mit einem Strippgas, z.B. in einem dem Reaktor vorgeschalteten Vorlagebehälter. Beispielsweise könnte als Strippgas Inertgas, insbesondere Stickstoff, Argon, CO₂, NO, Mischungen daraus, von oben oder von unten im Vorlagebehälter (bevorzugt von oben) über eine Fritte in den Behälter dem Pyrolysegut zu geführt werden, um so das Sauerstoffgas auszutreiben.

Liegt die polymere Verbindung in Form eines Schaums, insbesondere als Polyurethanweichschaum, vor wird der Schaum durch Pressen oder Heißpressen (200 bar) in seinem Volumen stark reduziert und kann danach zu kleinen Partikeln geschnitten oder geschreddert werden. Anschließend kann der auf solche Weise prozessierte Schaum leichter in den Pyrolysereaktor eingebracht werden.

Im Rahmen einer weiteren bevorzugten Ausführungsform des Verfahrens beträgt der absolute Druck in Schritt (b) höchstens 1,2 bar.

Im Rahmen einer weiteren bevorzugten Ausführungsform des Verfahrens beträgt der absolute Druck in Schritt (b) mindestens 0,8 bar.

Es hat sich ferner gezeigt, dass es im Allgemeinen noch vorteilhafter ist, die Verfahrensführung derart zu gestalten, dass die Temperatur des Schrittes (b) nicht über 300°C eingestellt ist. Im Rahmen einer weiteren bevorzugten Ausführungsform des erfindungsgemäßen Verfahrens beträgt daher die Temperatur im Schritt (b) zwischen 200°C und 300°C, weiter bevorzugt von 230°C bis 295°C, besonders bevorzugt von 250°C bis 290°C.

Im Rahmen einer weiteren bevorzugten Ausführungsform des erfindungsgemäßen Verfahrens beträgt im Schritt (b) die Menge an Sauerstoffgas im Reaktor höchstens 0,5 Vol.%, bevorzugt höchstens 0,1 Vol.%, jeweils bezogen auf das Gesamtvolumen der im Reaktor befindlichen Gase.

Im Rahmen einer ganz bevorzugten Ausführungsform des erfindungsgemäßen Verfahrens beträgt im Schritt (b) erstens die Temperatur zwischen 200°C und 300°C, weiter bevorzugt von 230°C bis 295°C, besonders bevorzugt von 250°C bis 290°C, und beträgt zweitens die Menge an Sauerstoffgas im Reaktor höchstens 0,5 Vol.%, bevorzugt höchstens 0,1 Vol.%, jeweils bezogen auf das Gesamtvolumen der im Reaktor befindlichen Gase. Dabei ist es wiederum äußerst bevorzugt, wenn zusätzlich drittens der absolute Druck in Schritt (b) von 0,8 bis 1,2 bar beträgt.

Eine bevorzugte Ausführungsform des Verfahrens liefert eine kontinuierliche Prozessführung. Dafür laufen zumindest die Schritte (a) und (b) im Rahmen einer kontinuierlichen Prozessführung gleichzeitig ab.

Das gemäß Schritt (c) erhaltene Pyrolyseprodukt kann mit gängigen Trennungsmethoden, beispielsweise Destillation oder selektive Kondensation, aufgearbeitet werden und dadurch (i) erhalten werden.

Es hat sich gezeigt, dass im herausgeführten Pyrolyserückstand eine große Menge an Verbindungen enthalten ist, die auf die in der ursprünglichen polymeren Verbindung des Pyrolysegutes enthaltenen weiteren Struktureinheiten, insbesondere der Polyol-Struktureinheiten, zurückzuführen sind. Im Rahmen eines erfindungsgemäß bevorzugten, sequentiellen Pyrolyseverfahrens erwies es sich als vorteilhaft, wenn der herausgeführte Pyrolyserückstand zusätzlich einer zweiten pyrolytischen Zersetzung bei einer Temperatur von mehr als 360°C unterworfen wird. Eine bevorzugte Ausführungsform des erfindungsgemäßen Verfahrens ist somit dadurch gekennzeichnet, dass in einem weiteren Schritt der aus dem Reaktor herausgeführte Pyrolyserückstand in einen zweiten Reaktor eingebracht wird, und dort bei einer Temperatur von mehr als 360°C, unter Erhalt von in der Gasphase befindlichem zweitem Pyrolysat und von nicht in der Gasphase befindlichem zweiten Pyrolyserückstand zersetzt wird, wobei während dieser Zersetzung die Menge an Sauerstoffgas im Reaktor höchstens 2,0 Vol.% bezogen auf das Gesamtvolumen der im zweiten Reaktor befindlichen Gase beträgt, und wobei während dieser Zersetzung das zweite Pyrolysat aus dem Reaktor herausgeführt wird, und der besagte zweite Pyrolyserückstand aus dem Reaktor herausgeführt wird.

Dabei hat es sich als bevorzugt erwiesen, wenn die im Pyrolysegut der vorangegangenen Pyrolyse enthaltene polymere Verbindung durch Umsetzung zumindest von
i1) mindestens einer organischen Isocyanatverbindung, enthaltend mindestens zwei bis vier, insbesondere zwei Isocyanatgruppen bindend an eine Kohlenwasserstoffeinheit mit 2 bis 8 Kohlenstoffatomen, bevorzugt mit 3 bis 8 Kohlenstoffatomen; mit
i2) mindestens einer organischen Verbindung mit mindestens zwei Hydroxygruppen,
erhalten wurde. Alle vorgenannten bevorzugten Ausführungsformen dieser Umsetzung von i1) mit i2) gelten im Rahmen dieser Ausführungsform ebenso als bevorzugt geeignet.

Besonders bevorzugt wird in der vorgenannten sequenziellen Ausführungsform des Verfahrens die Zersetzung im zweiten Reaktor bei mindestens 360°C, insbesondere mehr als 400°C. Abhängig vom dem Katalysator und der Reaktor Führung kann hier entweder Propen und Ethen erhalten werden, was zu Propylenoxid bzw. Ethylenoxid umgesetzt werden kann oder zu einer aromatischen Verbindung, (bevorzugt zu einer aromatischen Kohlenwasserstoffverbindung mit 6 bis 10 Kohlenstoffatomen, ganz besonders bevorzugt zu Benzol, Toluol, Xylol oder Gemischen daraus) umgesetzt werden.

Das erfindungsgemäße Verfahren kann mit Hilfe einer entsprechend ausgestalteten Pyrolysevorrichtung durchgeführt werden. Ein weiterer Gegenstand der Erfindung ist daher die Pyrolysevorrichtung, sowie die Verwendung einer Pyrolysevorrichtung enthaltend mindestens eine Dosiervorrichtung zur Einspeisung von Pyrolysegut, mindestens einen heizbaren Reaktor für die Pyrolyse, und mindestens einen Pyrolysat-Kollektor, wobei
besagter heizbarer Reaktor für die Pyrolyse mindestens ein Heizelement enthält, das zur Temperierung des Reaktors auf eine Temperatur von 200°C bis 350°C verwendet werden kann, und mindestens einen Einlass für Pyrolysegut enthält und mindestens einen davon verschiedenen Auslass für Pyrolysat und mindestens einen davon verschiedenen Auslass für Pyrolyserückstand enthält; und
Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
mindestens ein Pyrolysat-Kollektor mindestens eine auf eine Temperatur unter 200°C temperierte Kühlvorrichtung enthält, die im besagten Kollektor zur Senkung der Temperatur des aus dem besagten Reaktor herausgeführten Pyrolysates auf weniger als 200°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält; und
mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können,
zur Gewinnung von organischer Verbindung mit mindestens einer Aminogruppe durch Pyrolyse von polymerer Verbindung mit mindestens einer Polyurethan-Struktureinheit der Formel (I), 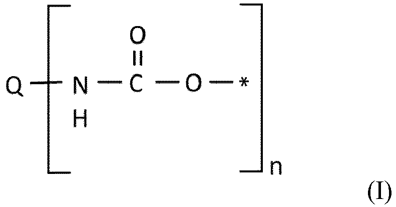 worin
Q für einen Kohlenwasserstoffrest mit 2 bis 8 Kohlenstoffatomen, insbesondere 3 bis 8 Kohlenstoffatomen, und
n für eine Zahl von 2 bis 4, bevorzugt von 2, steht
und -* eine kovalente Bindung zum Polymerrückgrat bedeutet.

Als bevorzugte polymere Verbindungen für die Pyrolyse gelten ebenfalls die im Rahmen der Beschreibung des Verfahrens genannten polymeren Verbindungen.

Es ist erfindungsgemäß bevorzugt, die besagte Pyrolysevorrichtung zur Rückgewinnung von mindestens einer aromatischen Aminoverbindung mit mindestens einer Aminogruppe, insbesondere ausgewählt aus Anilin, Toluidin, TDA, oder Mischungen daraus, zu verwenden.

Als Heizung des heizbaren Reaktors der besagten Pyrolysevorrichtung kann beispielsweise ein Heizelement, z.B. Heizwendel oder Heizplatten, dienen oder eine Vorrichtung zur Beheizung eines Gasstromes und zur Einbringung des beheizten Gasstroms in den Reaktor.

Der Reaktor enthält im Rahmen einer bevorzugten Ausführungsform zusätzlich mindestens einen Anschluss zu einer Gasquelle, wobei über eine Regelung, z.B. ein Ventil, ein Gasstrom im Reaktor, bevorzugt mit einer Fließgeschwindigkeit als Leerrohrgeschwindigkeit von zwischen 0,01 m/s bis 20 m/s, durch den Reaktor in den Pyrolysat-Kollektor fließt. Sollte als Reaktor ein Festbettreaktor gewählt werden, ist es erfindungsgemäß bevorzugt, wenn die Fließgeschwindigkeit des Gasstromes im Reaktor als Leerrohrgeschwindigkeit im Bereich von 0,03 m/s bis 1 m/s liegt. Sollte als Reaktor ein Wirbelbettreaktor gewählt werden, ist es erfindungsgemäß bevorzugt, die Fließgeschwindigkeit des Gasstromes im Reaktor als Leerrohrgeschwindigkeit im Bereich von 0,5 m/s bis 2 m/s liegt. Sollte als Reaktor ein Flugstromreaktor gewählt werden, ist es erfindungsgemäß bevorzugt, die Fließgeschwindigkeit des Gasstromes im Reaktor als Leerrohrgeschwindigkeit im Bereich von 5 m/s bis 20 m/s liegt. Der Gasstrom aus der Gasquelle kann beispielsweise vor der Einbringung in den Reaktor wie zuvor beschrieben durch die Heizung beheizt werden.

Der Pyrolysat-Kollektor der Pyrolysevorrichtung enthält bevorzugt eine Kühlvorrichtung, die im besagten Kollektor zur Senkung der Temperatur des aus dem besagten Reaktor herausgeführten Pyrolysates auf weniger als 50°C (weiter bevorzugt auf weniger als 30°C) unter Bildung von Pyrolyseprodukt verwendbar ist. Hierfür eignen sich bevorzugt Kühlaggregate die nach dem Wärmetauscherprinzip arbeiten. Dabei kann der Pyrolysat-Kollektor als selektiver Kondensator für eine selektive Kondensation von im Pyrolysat enthaltenen Pyrolyseprodukt-Bestandteilen ausgerüstet sein.

Unter einer "Fluidverbindung" wird erfindungsgemäß ein Teil der Vorrichtung verstanden, der Anlagenteile miteinander verbindet und durch die sich ein Stoff, der in jedem Aggregatzustand vorliegen kann, von einem Anlagenteil zum nächsten transportieren lässt, beispielsweise eine Zuleitung in Form eines Rohres.

Ferner ist es im Rahmen einer weiteren Ausführungsform der Pyrolysevorrichtung bevorzugt, wenn diese Pyrolysevorrichtung zusätzlich enthält,
mindestens einen weiteren heizbaren Reaktor für die Pyrolyse, der mindestens ein Heizelement enthält, das zur Temperierung des Reaktors auf eine Temperatur von über 360°C verwendet werden kann, und mindestens einen Einlass für Pyrolysegut enthält und mindestens einen davon verschiedenen Auslass für Pyrolysat enthält; und
mit der Maßgabe, dass der Auslass von Pyrolyserückstand des besagten heizbaren Reaktors in Fluidverbindung mit dem Einlass für Pyrolysegut des weiteren heizbaren Reaktors steht.

Im Rahmen dieser Ausführungsform ist es bevorzugt, wenn die Pyrolysevorrichtung mindestens einen weiteren Pyrolysatkollektor enthält der mit dem weiteren heizbaren Reaktor in Fluidverbindung steht, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist.

Auch besagter weiterer Pyrolysat-Kollektor ist bevorzugt derart ausgestaltet, dass er mindestens eine auf eine Temperatur unter 200°C temperierte Kühlvorrichtung enthält, die im besagten Kollektor zur Senkung der Temperatur des aus dem besagten Reaktor herausgeführten Pyrolysates auf weniger als 200°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält.

### Beispiele

a) Bereitstellung des Pyrolysegutes: Es wurde ein Polyurethanweichschaum mittels Standardverfahren aus den Komponenten gemäß Tabelle 1 hergestellt.

**Tabelle 1: Komponenten des Weichschaumes:**

| **Bestandteile** | **Gew.-%** |
|---|---|
| Polyol mit OH Zahl 48 | 64,26 |
| Wasser | 2,44 |
| Stabilisator | 0,64 |
| Farbstoff | 0,32 |
| Katalysatormischung aus Zinnoctanoat, 1,4-Diazabicyclo[2.2.2]octan und Bis(2-(N,N-dimethylamino)ethyl)ether | 0,16 |

| **Isocyanat** | |
|---|---|
| Desmodur^{®} T80¹ | 32.18 |

| | |
|---|---|
| ¹ Isocyanat der Firma Covestro Deutschland AG, 2,4- und 2,6-Toluylendiisocyanat (TDI) im Verhältnis von 80 : 20 | |

Zur Bereitstellung von Pyrolysegut wurde der Weichschaum mit einer Heizpresse bei 150 °C auf 1/20 seiner ursprünglichen Höhe verpresst und dann in 1 mm große Stücke geschnitten. Je 2 g dieser Stücke wurden anschließend in 5 Al₂O₃ Tiegeln mit Halterung in den Reaktor eingebracht. Die Pyrolyse wurde wie unter b) beschrieben durchgeführt.
b) Durchführung der Pyrolyse: Die Pyrolyse des Polyurethan Weichschaums, wurde in einem mit Ar durchströmten Festbettreaktor mit einem Volumen von 25 ml bei 273 °C durchgeführt.

Die Fließgeschwindigkeit des Stickstoff Gasstroms (Leerrohrgeschwindigkeit) im Reaktor betrug 0,07 m/s. Das präparierte Pyrolysegut wurde in den Reaktor eingebracht. Die Verweilzeit des eingebrachten Polyurethan-Materials betrugt 30 min. Der Reaktor wurde auf 273 °C aufgeheizt und dann 30 min bei dieser Temperatur gehalten. Hinter dem Reaktor befanden sich 2 Kondensatoren, um die Flüssigkomponenten aus dem entstehenden Pyrolysegas abzutrennen. Der entstehende Koksanteil wurde durch Wiegen der Al₂O₃ Tiegel nach der Pyrolyse bestimmt. Das Gas nach den 2 Kondensatoren wurde mittels online GC charakterisiert. Die Komponenten im als Öl anfallendem Pyrolyseprodukt wurden mit GC-FID bestimmt. Dazu wurde ein Agilent 7890A mit einer Säule SupelcoSPB 50 benutzt. Das Pyrolyse Öl wurde mit Aceton 1:50 oder 1: 100 verdünnt. Die Ergebnisse werden in Tabelle 2 illustriert.

Der aus dieser Pyrolyse erhaltene Pyrolyserückstand wurde erneut pyrolysiert. Diese zweite Pyrolyse wurde in einem mit N₂ durchströmten Festbettreaktor mit einem Volumen von 25 ml bei 360 °C durchgeführt.

Die Fließgeschwindigkeit des Stickstoff Gasstroms (Leerrohrgeschwindigkeit) im Reaktor betrug 0,07 m/s. Der Pyrolyserückstand der vorherigen Pyrolyse wurde als Pyrolysegut in den Reaktor eingebracht. Die Verweilzeit des eingebrachten Pyrolysegutes betrugt 30 min. Der Reaktor wurde auf 360°C aufgeheizt und dann 30 min bei dieser Temperatur gehalten. Hinter dem Reaktor befanden sich 2 Kondensatoren, um die Flüssigkomponenten aus dem entstehenden Pyrolysegas abzutrennen.

**Tabelle 2: Zusammensetzung des erhaltenen Pyrolyseproduktes, des Restgases und des Pyrolyserückstandes aus der Pyrolyse des Polyurethanweichschaums bei 273°C [Gew.-%]:**

| **Pyrolyse** | **Gew.-%** |
|---|---|
| **Pyrolyseprodukt (ÖI):** | |
| TDA | 14,62 |
| Restöl (nicht charakterisiert) | 11,90 |

| **Gasphase nach Kondensation** | |
|---|---|
| CO₂ | 4,00 |
| CO | 0,04 |
| Propen | 3,27 |
| weitere Gase | 2,51 |

| **Pyrolyserückstand** | |
|---|---|
| Polyol mit Koks | 63,66 |
| **Gesamt** | 100,00 |

## Patentansprüche

1. Verfahren zur Pyrolyse, umfassend mindestens die folgenden Schritte
(a) Einbringung von Pyrolysegut, enthaltend mindestens eine polymere Verbindung mit mindestens einer Polyurethan-Struktureinheit der Formel (I), 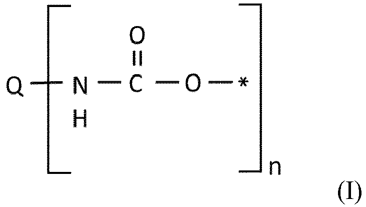 worin
Q für einen Kohlenwasserstoffrest mit 2 bis 8 Kohlenstoffatomen, bevorzugt 3 bis 8 Kohlenstoffatomen, und
n für eine Zahl von 2 bis 4, besonders bevorzugt von 2, steht
und -* eine kovalente Bindung zum Polymerrückgrat bedeutet,
in einen Reaktor;
(b) Zersetzung zumindest der in Schritt (a) eingebrachten polymeren Verbindung des Pyrolyseguts in dem Reaktor bei einer Temperatur von 200°C bis 350°C, unter Erhalt von in der Gasphase befindlichem Produkt als Pyrolysat und von nicht in der Gasphase befindlichem Pyrolyserückstand, wobei
(i) während der besagten Zersetzung die Menge an Sauerstoffgas im Reaktor höchstens 2,0 Vol.% bezogen auf das Gesamtvolumen der im Reaktor befindlichen Gase beträgt, und
(ii) während der besagten Zersetzung das Pyrolysat aus dem Reaktor herausgeführt wird, und
(iii) der besagte Pyrolyserückstand aus dem Reaktor herausgeführt wird;
(c) Abkühlung des herausgeführten Pyrolysates auf eine Temperatur von weniger als 200°C unter Erhalt von Pyrolyseprodukt, ausgewählt aus Pyrolysatkondensat, Pyrolysatsublimat oder einer Mischung daraus;
(d) optional Aufarbeitung des Pyrolyseprodukts.

2. Verfahren nach einem der vorangehenden Ansprüche, **dadurch gekennzeichnet, dass** das eingebrachte Pyrolysegut auf 200°C bis 350°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes 1 Sekunde bis 5 Stunden, bevorzugt zwischen 2 Minuten und 120 Minuten, beträgt.

3. Verfahren nach einem der Ansprüche 1 oder 2, **dadurch gekennzeichnet, dass** die Temperatur im Schritt (b) zwischen 200°C und 300°C, von 230°C bis 295°C, bevorzugt 250°C bis 290°C beträgt.

4. Verfahren nach einem der vorangehenden Ansprüche, **dadurch gekennzeichnet, dass** der absolute Druck in Schritt (b) höchstens 1,2 bar beträgt.

5. Verfahren nach einem der vorangehenden Ansprüche, **dadurch gekennzeichnet, dass** der absolute Druck in Schritt (b) mindestens 0,8 bar beträgt.

6. Verfahren nach einem der vorangehenden Ansprüche, **dadurch gekennzeichnet, dass** die Herausführung des Pyrolysates aus dem Reaktor durch einen durch den Reaktor geführten Gasstrom oder durch Sog gewährleistet wird und bevorzugt dadurch eine Verweildauer des Pyrolysates als Zeitraum zwischen dem Zeitpunkt der Bildung des Pyrolysats und dem Zeitpunkt der Ausbringung des resultierenden Pyrolysates aus dem Reaktor 0,1 Sekunden bis 600 Sekunden, bevorzugt zwischen 0,5 Sekunden und 300 Sekunden, besonders bevorzugt 0,5 Sekunden bis 200 Sekunden, beträgt.

7. Verfahren nach einem der vorangehenden Ansprüche, **dadurch gekennzeichnet, dass** die Herausführung des Pyrolysates aus dem Reaktor durch einen durch den Reaktor geführten Gasstrom gewährleistet wird, dessen Fließgeschwindigkeit im Reaktor als Leerrohrgeschwindigkeit im Bereich von 0,01 m/s bis 20 m/s liegt.

8. Verfahren nach einem der vorangehenden Ansprüche, **dadurch gekennzeichnet, dass** zumindest die Schritte (a) und (b) im Rahmen einer kontinuierlichen Prozessführung gleichzeitig ablaufen.

9. Verfahren nach einem der vorangehenden Ansprüche, **dadurch gekennzeichnet, dass** im Schritt (b) die Menge an Sauerstoffgas im Reaktor höchstens 0,5 Vol.%, bevorzugt höchstens 0,1 Vol.%, jeweils bezogen auf das Gesamtvolumen der im Reaktor befindlichen Gase beträgt.

10. Verfahren nach einem der vorangehenden Ansprüche, **dadurch gekennzeichnet, dass** der mit besagten Pyrolysegut befüllte Reaktor mit Inertgas, insbesondere mit Stickstoff, Argon, CO₂, NO oder Mischungen daraus durchströmt wird.

11. Verfahren nach einem der vorangehenden Ansprüche, **dadurch gekennzeichnet, dass** die polymere Verbindung mindestens eine Polyurethan-Struktureinheit der Formel (Ia) enthält 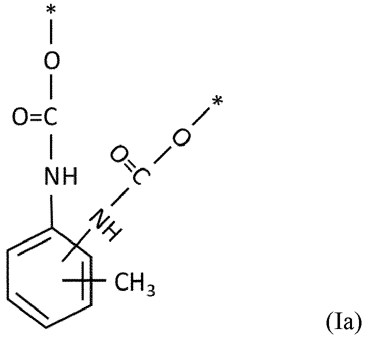 worin
-* für eine kovalente Bindung zum Polymerrückgrat steht.

12. Verfahren nach einem der vorangehenden Ansprüche, **dadurch gekennzeichnet, dass** die polymere Verbindung durch Umsetzung zumindest von
i1) mindestens einer organischen Isocyanatverbindung, enthaltend mindestens zwei bis vier, insbesondere zwei Isocyanatgruppen bindend an eine Kohlenwasserstoffeinheit mit 2 bis 8 Kohlenstoffatomen, bevorzugt mit 3 bis 8 Kohlenstoffatomen; mit
i2) mindestens einer organischen Verbindung mit mindestens zwei Hydroxygruppen,
erhältlich ist.

13. Verfahren nach Anspruch 12, **dadurch gekennzeichnet, dass** die mindestens eine organische Isocyanatverbindung als besagte Kohlenwasserstoffeinheit eine Einheit enthält, die die zuvor genannte Anzahl an Kohlenstoffatomen aufweist und sich ableitet von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohlenwasserstoffeinheiten.

14. Verfahren nach einem der Ansprüche 12 oder 13, **dadurch gekennzeichnet, dass** als organische Polyisocyanatkomponente mindestens ein Diisocyanat der Formel (III) ausgewählt wird,

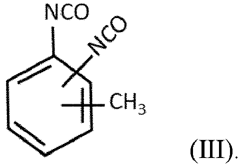

15. Verfahren nach einem der vorangehenden Ansprüche, **dadurch gekennzeichnet, dass** besagte polymere Verbindung als Bestandteil eines Materials in Form festförmiger Teilchen, insbesondere in Form eines körnigen Gemenges, in den Reaktor eingebracht wird.

16. Verfahren nach einem der vorangehenden Ansprüche, **dadurch gekennzeichnet, dass** in einem weiteren Schritt der aus dem Reaktor herausgeführte Pyrolyserückstand in einen zweiten Reaktor eingebracht wird, und dort bei einer Temperatur von mehr als 360°C, unter Erhalt von in der Gasphase befindlichem zweitem Pyrolysat und von nicht in der Gasphase befindlichem zweiten Pyrolyserückstand zersetzt wird, wobei während dieser Zersetzung die Menge an Sauerstoffgas im Reaktor höchstens 2,0 Vol.% bezogen auf das Gesamtvolumen der im zweiten Reaktor befindlichen Gase beträgt, und wobei während dieser Zersetzung das zweite Pyrolysat aus dem Reaktor herausgeführt wird, und der besagte zweite Pyrolyserückstand aus dem Reaktor herausgeführt wird.

17. Verfahren nach Anspruch 16 oder 17, **dadurch gekennzeichnet, dass** das zweite Pyrolysat einer Aufarbeitung unterworfen und das resultierende Produktgas einem dritten Reaktor zugeführt und dort zu Alkylenoxid umgesetzt wird.

18. Verwendung einer Pyrolysevorrichtung enthaltend mindestens eine Dosiervorrichtung zur Einspeisung von Pyrolysegut, mindestens einen heizbaren Reaktor für die Pyrolyse, und mindestens einen Pyrolysat-Kollektor, wobei
besagter heizbarer Reaktor für die Pyrolyse mindestens ein Heizelement enthält, das zur Temperierung des Reaktors auf eine Temperatur von 200°C bis 350°C verwendet werden kann, und mindestens einen Einlass für Pyrolysegut enthält und mindestens einen davon verschiedenen Auslass für Pyrolysat enthält; und
Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
mindestens ein Pyrolysat-Kollektor mindestens eine auf eine Temperatur unter 200°C temperierte Kühlvorrichtung enthält, die im besagten Kollektor zur Senkung der Temperatur des aus dem besagten Reaktor herausgeführten Pyrolysates auf weniger als 200°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält; und
mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können,
zur Gewinnung von organischer Verbindung mit mindestens einer Aminogruppe durch Pyrolyse von polymerer Verbindung mit mindestens einer Polyurethan-Struktureinheit der Formel (I), 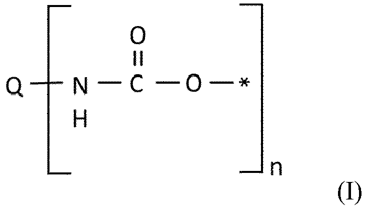 worin
Q für einen Kohlenwasserstoffrest mit 3 bis 8 Kohlenstoffatomen und n für eine Zahl von 2 bis 4, bevorzugt für 2, steht und -* eine kovalente Bindung zum Polymerrückgrat bedeutet.
